# EUROPÄISCHE PATENTANMELDUNG

(11) **EP 1 811 015 A1**
(43) Veröffentlichungstag der Anmeldung: **25.07.2007**
(21) Anmeldenummer: 07100839.5
(22) Anmeldetag: 19.01.2007
(51) Int. Cl.: C12M 1/04, C12M 1/107, C02F 11/02

(54) **Einleitung von Biogas in einen Fermenter**

(30) Priorität: 20.01.2006 DE 102006002836
(71) Anmelder: Eckard, Horst K., 36169 Rasdorf (DE); Grimm, Arnold, 36154 Hosenfeld (DE); Hemschemeier, Hans, 51647 Gummersbach (DE)
(72) Erfinder: Grimm, Arnold, 36154, Hosenfeld (DE); Hemschemeier, Hans, 51647, Gummersbach (DE)
(74) Vertreter: Schwabe - Sandmair - Marx

(57) **Zusammenfassung**

Biogasanlage zur Erzeugung von Biogas aus einem Ferment, das wenigstens eines aus landwirtschaftlichem Abfall, Schlachtabfall, Silage oder Energiepflanzen enthält, die Biogasanlage umfassend
a) einen Fermenter (1) für einen mikrobiellen Abbau des Ferments zur Erzeugung von Biogas,
b) und ein Einleitsystem mit einer Vielzahl von Düsen (13) für das Einblasen von Biogas in das Ferment,
c) wobei die Düsen (13) über zumindest einen größeren Teil eines Bodens (1a) des Fermenters (1) verteilt angeordnet und wenigstens fünf Düsen (13) pro Quadratmeter des betreffenden Teils des Bodens vorgesehen sind.

## Beschreibung

Die Erfindung betrifft eine Biogasanlage und ein Verfahren zur Erzeugung von Biogas. Zur Intensivierung der Fermentation wird Biogas in einen Fermenter eingeblasen, um ein darin enthaltenes Ferment mit dem Biogas zu durchmischen.

Üblich ist die Durchmischung des Ferments mit mechanischen Rührwerken. Mechanische Rührwerke haben allerdings einen hohen Energiebedarf und erfordern eine stabile Statik des Fermenters und verursachen daher hohe Kosten. Aufgrund hohen mechanischen Verschleißes müssen die Schraubenblätter der Rührwerke in regelmäßigen Wartungsintervallen ausgetauscht werden. Die hierdurch verursachten Kosten haben schon zu Versuchen geführt, die Schraubenblätter mit einem keramischen Überzug zu schützen. Dies trieb die Kosten weiter hoch, gleichzeitig wurde die Wirtschaftlichkeit einer damit bestückten Biogasanlage weiter gesenkt.

Es ist eine Aufgabe der Erfindung, auf preiswerte und störungssichere Weise die Fermentation in einem Fermenter zu intensivieren.

Die Erfindung betrifft eine Biogasanlage mit einem Fermenter für einen mikrobiellen Abbau eines Ferments zur Erzeugung von Biogas und einem Einleitsystem, das eine Vielzahl von Düsen für die Einleitung von Biogas in das Ferment umfasst. Der Fermenter weist einen Boden und um eine Hochachse umlaufend eine von dem Boden aufragende Seitenwandung auf. Die Seitenwandung kann polygonal, insbesondere wie in der deutschen Patentanmeldung Nr. 10 2005 049 476,5 beschrieben, oder rund sein, vorzugsweise kreisrund. Ein polygonaler Grundriss wird wie in dieser Anmeldung beschrieben im Hinblick auf den günstigen Preis bevorzugt. Besonders bevorzugt wird ein Grundriss mit wenigstens sechs gleichlangen Seiten. Die ältere genannte Anmeldung wird hinsichtlich der Konstruktion des Fermenters in Bezug genommen.

Nach der Erfindung sind die Düsen zumindest über einen größeren Teil der Gesamtfläche des Bodens verteilt angeordnet. Die Flächenbelegung beträgt wenigstens fünf Düsen pro Quadratmeter des betreffenden Teils des Bodens, vorzugsweise beträgt sie in dem betreffenden Teil wenigstens zehn oder wenigstens zwanzig Düsen pro Qudratmeter. Als der größere Teil wird ein Anteil an der Gesamtfläche von wenigstens 30% verstanden. Bevorzugt sind die Düsen in der genannten Mindestflächenbelegung über wengistens den überwiegenden Teil der Gesamtfläche des Bodens verteilt angeordnet. Diesbezüglich gilt: je größer der Anteil der erfindungsgemäß mit Düsen belegten Fläche, desto besser. Ein Anteil von wenigstens 80%, am besten 100% der Bodengesamtfläche ist daher anzustreben. Mit den in großer Zahl und fein verteilter Anordnung vorhandenen Düsen wird das Ferment im Betrieb der Anlage von entsprechend vielen Düsenstrahlen durchsetzt, entsprechend groß ist die Kontaktfläche zwischen dem Ferment und dem eingeblasenen Biogas. Allerdings ist es vorteilhaft, wenn die Belegung im betreffenden Flächenteil nicht größer als etwa fünfzig bodennah angeordnete Düsen pro Quadratmeter beträgt.

In bevorzugter Ausführung umfasst das Einleitsystem eine schlauch- oder rohrförmige Bodenleitung, die auf oder bodennah über dem Boden des Fermenters angeordnet ist und einen Leitungsmantel aufweist, der mit den Düsen oder zumindest einem Teil der Düsen versehen ist. Vorzugsweise ist die Bodenleitung auf dem Boden abgestützt. Alternativ oder zusätzlich kann sie jedoch auch an der Seitenwandung abgestützt oder hängend angeordnet sein. Obgleich es in einer Abwandlung denkbar wäre, die Düsen integriert im Boden anzuordnen, ist die Anordnung auf oder über dem Boden insbesondere kostengünstiger sowohl im Hinblick auf die Herstellung des Bodens, der herkömmlich als geschlossene Fläche gebildet sein kann, als auch im Hinblick auf die Bereitstellung der Düsen. Ferner können bestehende Fermenter einfacher umgerüstet werden, beispielsweise von einem mechanischen Rührwerk auf ein erfindungsgemäßes Düsenfeld. Ferner kann der Boden als solcher wie bevorzugt überall flüssigkeitsdicht ausgeführt werden.
In einer einfachen ersten Variante liegt die Bodenleitung einfach auf dem Boden des Fermenters auf. In einer bevorzugten zweiten Variante ist sie in einem flachen Gestell aufgenommen, das auf dem Boden aufliegt, vorzugsweise lose aufliegt, und die Bodenleitung in einem geringen Abstand von wenigen Zentimetern, vorzugsweise höchstens fünfzig, bevorzugter höchstens dreißig Zentimetern, über dem Boden hält. Die Austrittsachsen eines Teils der Düsen können in Richtung auf den Boden weisen und vorteilhafterweise so gerichtet sein, dass auf dem Boden ein gewisser Fegeeffekt erzeugt wird. Die Düsen können beispielsweise so angeordnet sein, dass sie in der Art einer Perlenkette angeordnet sind, die sich fortgesetzt um die Längsachse oder Mittellängslinie der Bodenleitung windet.

Die Bodenleitung kann einen oder mehrere Knotenbereiche und vom jeweiligen Knotenbereich abzweigende Leitungsäste umfassen, durch die das Biogas ausgehend von dem Knotenbereich oder den gegebenenfalls mehreren Knotenbereichen verteilt und über in den Leitungsästen gebildete Düsen ausgeblasen wird. In bevorzugten Ausführungen ist die Bodenleitung auf oder über den Boden in mehreren Schleifen um eine einzige oder mehrere voneinander beabstandete Hochachsen des Fermenters gewunden. Vorzugsweise ist sie spiralig um eine zentrale Hochachse oder mäanderförmig um mehrere Hochachsen oder gegebenenfalls jeweils spiralig um mehrere Hochachsen gewunden. Vorteilhaft ist auch bereits eine einzige Schleife, die im Bereich eines äußeren Umfangsrands des Bodens, d.h. nahe der Seitenwandung, über einen zumindest überwiegenden Teil des Umfangsrands, vorzugsweise umlaufend, verläuft. Vorteilhafter ist in derartigen Ausführungen allerdings, wenn zusätzlich zu der randnahen Leitung wenigstens eine weitere Schleife in einen zentralen Bereich des Fermenters angeordnet ist. Vorzugsweise bildet ein einziger Schlauch oder ein einziges Rohr die bevorzugt mehreren Schleifen, d.h. der Schlauch oder das Rohr setzt sich kontinuierlich von einem stromaufwärtigen Ende der Schleife oder Schleifen bis zu deren stromabwärtigen Ende fort. Grundsätzlich sind auch Mischformen mit einer oder mehreren Verzweigungen und gewundenen Leitungsästen denkbar. Nicht zuletzt aus Kostengründen wird jedoch der Verwendung eines einzigen Schlauchs oder Rohrs, der oder das mäanderförmig oder vorzugsweise spiralig gewunden den größeren Teil der Gesamtfläche des Bodens bedeckt oder überdeckt, der Vorzug gegeben. Die Bodenleitung kann sich zwar grundsätzlich auch insgesamt mit einer Neigung oder abschnittsweise geneigt oder in eine Richtung senkrecht zu dem Boden erstrecken, vorzugsweise verläuft sie jedoch überall zumindest im Wesentlichen parallel zu dem Boden, der vorzugsweise horizontal erstreckt ist. Die Schleifen bzw. Windungen sollten voneinander einen Abstand von wenigstens zwanzig Zentimetern und höchstens einem Meter, vorzugsweise von 40 ± 20 cm haben.

Falls wie bevorzugt ein biegbarer Schlauch die Bodenleitung bildet, sollte der Schlauch in sich doch so fest sein, dass er unter dem Druck des Ferments nicht in sich kollabiert, wenn durch den Schlauch kein Biogas gedrückt wird.

Die Bodenleitung wird vorzugsweise über eine schlauch- oder rohrförmige, zumindest auch vertikal erstreckte Vertikalleitung mit dem Biogas versorgt. Bevorzugt erstreckt sich die Vertikalleitung zumindest im wesentlichen in die vertikale Richtung, grundsätzlich kann sie jedoch auch schräg verlaufen oder schräg zur Vertikalen verlaufende Abschnitte aufweisen. Die Vertikalleitung kann in einem Randbereich des Fermenters, beispielsweise an der Seitenwand angeordnet sein, bevorzugter ist sie jedoch zentral angeordnet, besonders bevorzug koaxial zu der Seitenwandung. Die Vertikalleitung kann mit der Bodenleitung in einem Stück geformt sein, insbesondere als ein einziger Schlauch. Sie kann aber auch separat von der Bodenleitung gefertigt und mittels geeigneter Fügetechnik mit der Bodenleitung gasdicht verbunden sein. Bevorzugt wird die Bodenleitung nur über eine einzige Vertikalleitung mit dem Biogas versorgt. Gegebenfalls kann es jedoch auch von Vorteil sein, mehrere Vertikalleitungen vorzusehen, über die das Biogas an mehreren Orten in die Bodenleitung geleitet wird. Es können auch mehrere separate, d.h. nicht miteinander verbundene Bodenleitungen mit je wenigstens einer, vorzugsweise genau einer als Zuleitung dienenden Vertikalleitung vorgesehen sein. Mehrere Vertikalleitungen mit einer einzigen oder mehreren separaten Bodenleitungen können vor allem bei großen Fermentern vorteilhaft sein. Ein primäres Anwendungsgebiet der Erfindung sind allerdings kleinere und mittlere Anlagen, wie sie in landwirtschaftlichen Betrieben Verwendung finden.

In dem Fermenter wird vorzugsweise frisches Substrat aus biologischem Material fermentiert. In bevorzugten einfachen Ausführungen umfasst die Biogasanlage nur eine Stufe der Fermentation, kann aber durchaus mehrere Fermenter je mit einem erfindungsgemäßen Einleitsystem umfassen, in denen je die gleiche Fermentation durchführbar ist.

Die Erfindung zielt in erster Linie auf Fermenter ab, deren Innenvolumen einen Querschnitt von höchstens 80 qm, bevorzugter höchstens 40 qm, und eine Höhe von vorzugsweise höchstens 20 m, bevorzugter höchstens 10 m aufweist. Der Innenquerschnitt ist über die Höhe des Innenvolumens vorzugsweise zumindest im wesentlichen konstant. Besonders bevorzugt werden Fermenter mit einem Innenvolumen, dessen Höhe aus dem Bereich von 4 bis 10 m und dessen Querschnitt aus dem Bereich von 15 bis 30 qm gewählt ist. So kann ein Fermenter mit Kreisquerschnitt insbesondere einen Durchmesser von 4 bis 7 m und auch eine Höhe von 4 bis 7 m haben. Im bevorzugt polygonalen Querschnitt beträgt der Abstand zwischen einander gegenüberliegenden Seitenwänden ebenfalls bevorzugt wenigstens 4 und höchstens 7 m, und auch die Höhe beträgt bevorzugt mindestens 4 und höchstens 7 m.

Die wenigstens eine Vertikalleitung oder die gegebenenfalls mehreren Vertikalleitungen ist oder sind an ihrem Mantel vorteilhafterweise ebenfalls mit einer Vielzahl von Düsen versehen, bevorzugt zumindest in einem unteren Abschnitt der Vertikalleitung oder jeder der Vertikalleitungen. Eine oder mehrere derartige Vertikalleitungen können grundsätzlich sogar die Bodenleitung ersetzen, bevorzugter unterstützen sie mit ihren Düsen jedoch nur das Düsenfeld der Bodenleitung und versorgen wie bereits erwähnt die Bodenleitung oder mehreren Bodenleitungen mit dem Biogas. Ein Einblasen von Biogas über die Bodenleitung und die Vertikalleitung in Kombination ist besonders vorteilhaft, da die Düsen der Bodenleitung das Biogas von unten nach oben und die Düsen der Vertikalleitung Biogas in das von unten aufsteigende Biogas von der Seit her einblasen können, so dass eine zusätzliche Verwirbelung erzeugt werden kann.

Die Düsen der Bndenleitung(en) können zwar grundsätzlich zueinander parallele Strahlaustrittsachsen aufweisen, bevorzugt weisen jedoch die Strahlaustrittsachsen einer ersten Gruppe von Düsen winkelig zu den Strahlaustrittsachsen einer zweiten Gruppe von Düsen, und noch bevorzugter weisen auch Strahlaustrittsachsen von Düsen der gleichen Gruppe winkelig zueinander. Dabei können die Strahlaustrittsachsen in Bezug auf ihre Achsrichtungen in gewisser Weise geordnet sein, nämlich so, dass das austretende Biogas dem Ferment einen Drehimpuls erteilt. Hierfür ist es vorteilhaft, wenn mehrere der Austrittsachsen, die in räumlicher Nähe zueinander angeordnet sind, eine gemeinsame Achse in einem Abstand kreuzen und so ausgerichtet sind, dass sie um die betreffende Achse einen Drehimpuls erzeugen. Die gemeinsame Achse kann insbesondere eine Längsachse der Bodenleitung sein. Falls die Bodenleitung wie bevorzugt gewunden ist, ersetzt die gewundene Mittellängslinie des Schlauchs oder Rohrs die betreffende Achse. In ebenfalls bevorzugten Ausführungen weisen die Strahlaustrittsachsen oder ein Teil der Strahlaustrittsachsen aber einfach nur radial zu der Leitung, aus der sie austreten.
Die Düsen haben vorteilhafterweise einen Strömungsquerschnitt von wenigstens 0.2 und höchstens 10 mm². Bevorzugter sind die Düsenquerschnitte aus dem Bereich von 0.5 mm² und 3 mm² gewählt. Die Angaben gelten bei einer in Richtung der jeweiligen Austrittsachse variierenden Querschnittsfläche der Düse für den engsten Querschnitt, ansonsten für den über die Düsenlänge konstanten Querschnitt. Falls der Düsenquerschnitt kreisrund ist, wie dies bevorzugt wird, hat die besagte Querschnittsfläche einen Durchmesser von besonders bevorzugt 1 bis 1.2 mm, wenigstens jedoch 0.5 mm und höchstens 3 mm. Ein kreisrunder Querschnitt oder eine an den Kreis angenäherte Querschnittsform, beispielsweise ein Quadrat oder eine Ellipse werden bevorzugt.

Das hinsichtlich der Form, der Anordnung der Bodenleitung(en) und deren Düsen Gesagte gilt auch für bevorzugte Ausführungen der Vertikalleitung(en).

Die Bodenleitung hat einen Querschnitt von vorzugsweise wenigstens 5 cm² und vorzugsweise höchstens 200 cm², bevorzugter von wenigstens 7 cm² und höchstens 120 cm². Eine Querschnittsfläche aus dem unteren Bereich bis etwa 50 cm² wird bevorzugt. Im Falle einer Leitung mit kreisrundern Innenquerschnitt wird ein Innendurchmesser von vorzugsweise wenigstens 3 cm und vorzugsweise höchstens 15 cm, bevorzugter höchstens 12 cm gewählt. Die Werte für den Strömungsquerschnitt gelten bevorzugt auch für die Vertikalleitung(en).

Das Einleitsystem für das Biogas kann in Doppelfunktion auch zum Einleiten eines mikrobiell abbaubaren flüssigen Substrats verwendet werden. Das Substrat kann beispielsweise Gülle oder ein Gemisch von landwirtschaftlichen Abfällen mit oder ohne Gülle, sein. Falls über das Einleitsystem ein Gemisch aus Biogas und flüssigem Substrat eingepresst bzw. eingespritzt wird, haben die Düsen eine Querschnittsfläche von vorzugsweise wenigstens 5 cm² und vorzugsweise höchstens 20 cm². Bei kreisrundem Düsenquerschnitt wird ein Durchmesser der Düsen aus dem Bereich von 3 bis 4 mm besonders bevorzugt.

Die Düsen sind vorzugsweise Lochdüsen, bevorzugt mit kreisrundem Düsenquerschnitt.

Mit dem Biogas kann vorteilhafterweise nicht nur kinetische Energie in das Ferment eingetragen werden, sondern zusätzlich auch Wärme. Das eingepresste Biogas ist daher vorzugsweise wärmer als das Ferment. Die intensive Durchmischung mit der großen Kontaktfläche zwischen dem Biogas und dem Ferment ist auch für einen intensiven Wärmeaustausch günstig.

Das der Durchmischung dienende Biogas entstammt vorteilhafterweise dem Fermenter. Das im Fermenter durch den mikrobiellen Abbau erzeugte Gas kann unmittelbar, d.h. ohne weiteren Prozessschritten unterworfen zu sein, auf die erfindungsgemäße Art und Weise in das Ferment eingeblasen werden. Allenfalls wird es auf den hierfür erforderlichen Druck gebracht. In einer bevorzugten Alternative wird das Biogas jedoch einem dem Fermenter nachgeschalteten Gasspeicher entnommen und mittels einer Pumpe zurückgepumpt und auf den erforderlichen Druck komprimiert. Der Gasspeicher kann noch im Fermenter oder bereits außerhalb des Fermenters angeordnet sein. Die Biogasanlage verfügt in beiden Alternativen vorteilhafterweise über eine Drucksteuer- oder -regeleinrichtung für die Erzeugung eines Überdrucks gegenüber der in dem Fermenter stehenden Säule des Ferments. Das Biogas wird vorzugsweise mit einem Überdruck von wenigstens 0.5 bar in das Ferment geblasen, wobei der Überdruck am Ort der betreffenden Düse gemessen wird. Besonders günstig ist ein Überdruck von 1 bar ± 0.2 bar. Die Drucksteuer- oder -regeleinrichtung umfasst in bevorzugter Ausführung einen Kompressor zur Erzeugung des Überdrucks und Förderung des Biogases durch das Einleitsystem und ferner eine Druckeinstelleinrichtung für die Einstellung des Überdrucks.

Auch die Unteransprüche und deren Kombinationen beschreiben vorteilhafte Merkmale der Erfindung.

Neben der am besten flächendeckenden Belegung des Bodens mit Düsen enthält die Erfindung weitere vorteilhafte Merkmale, die auch ungeachtet der Frage der Düsenbelegung alleine von Vorteil sind, obgleich sie mit dem Merkmal der Düsenbelegung vorteilhaft zusammenwirken, So ist alleine bereits vorteilhaft die Anordnung eines Schlauchs oder Rohrs auf oder nahe über dem Boden des Fermenters, da eine solche Bodenleitung einfach herstellbar und verlegbar sowie ferner robust und im Ergebnis besonders preiswert ist. Die Anmelder behalten es sich daher vor, auf die Ausgestaltung der Bodenleitung als Rohr oder Schlauch, das oder der in wenigstens einer Schleife gewunden ist, eine Teilungsanmeldung zu richten. Besonders vorteilhaft ist in diesem Zusammenhang auch, dass die Bodenleitung sich an ihrem stromaufwärtigen Ende in die Versorgungsleitung, vorzugsweise Vertikalleitung, fortsetzen kann.

Nachfolgend werden Ausführungsbeispiele der Erfindung anhand von Figuren erläutert. An den Ausführungsbeispielen offenbar werdende Merkmale bilden je einzeln und in jeder Kombination die Gegenstände der Ansprüche und auch die vorstehend beschriebenen Ausgestaltungen vorteilhaft weiter. Es zeigen:
- Figur 1: eine Biogasanlage mit einem Fermenter, einem Gasspeicher und einem Einleitsystem für das Einblasen von Biogas in den Fermenter,
- Figur 2: eine Bodenleitung des Einleitsystems nach einem ersten Ausführungsbeispiel,
- Figur 3: eine Bodenleitung des Einleitsystems nach einem zweiten Ausführungsbeispiel,
- Figur 4: einen Abschnitt der Bodenleitung,
- Figur 5: eine Vertikalleitung des Einleitsystems,
- Figur 6: ein erstes Ausführungsbeispiel einer Substratzuführung und
- Figur 7: ein zweites Ausführungsbeispiel einer Substratzuführung.

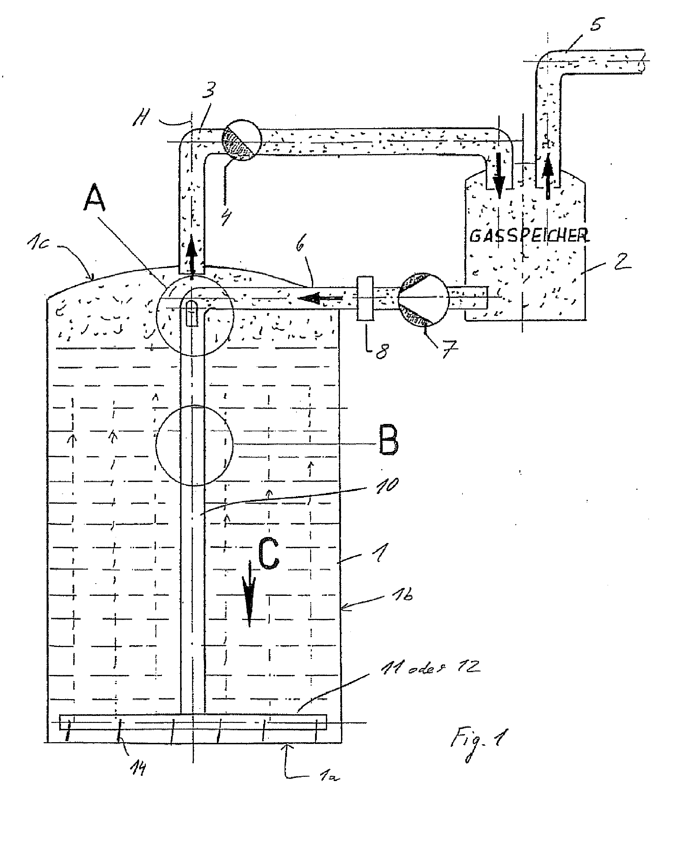

Figur 1 zeigt eine Biogasanlage mit einem Fermenter 1, einem dem Fermenter 1 zugeordneten Gassspeicher 2 und einem Einleitsystem, mittels dem Biogas aus dem Gasspeicher 2 in den Fermenter 1 zurückgeführt werden kann. In dem Fermenter 1 wird biologisch abbaubares Material mittels Mikroorganismen anaerob abgebaut und Biogas erzeugt. Um den mikrobiellen Abbau des biologischen Materials zu intensivieren und die Biogaserzeugung dadurch zu beschleunigen, wird ein Teil des erzeugten Biogases durch das Einleitsystem in das flüssige Ferment eingeblasen. Das Ferment ist die im Fermenter 1 enthaltene Mischung aus dem biologischen Material und den Mikroorganismen. Das Ferment kann insbesondere landwirtschaftlichen Abfall, Schlachtabfall, Silage oder Energiepflanzen einzeln oder in einem Gemisch enthalten.

Der Fermenter 1 weist einen Boden 1a, von dem Boden 1a aufragend und um eine zentrale Hochachse H des Fermenters 1 umlaufend eine Seitenwandung 1b und eine Decke 1c auf. In dem Fermenter 1 bildet das Ferment eine Säule. Zwischen der Säule und der Decke 1c hat sich ein Gasraum gebildet, aus dem Biogas über eine Verbindungsleitung 3 und einen Kondensatabscheiter 4 in den Gasspeicher 2 gelangt. Der größere Teil des im Gasspeicher 2 zwischengespeicherten Biogases wird über eine Abführleitung 5 einem Blockheizkraftwerk zugeführt, um aus dem Biogas elektrische und thermische Energie zu gewinnen. Ein demgegenüber kleinerer Teil des Biogases wird aus dem Biogasspeicher 2 über das Einleitsystem in das Ferment eingeblasen.

Das Einleitsystem umfasst eine aus dem Gasspeicher 2 in den Fermenter 1 führende Zuführleitung 6. Die Zuführleitung 6 verläuft durch den Gasraum des Fermenters 1 und anschließend als Vertikalleitung 10 durch das Ferment vertikal nach unten bis nahe zu dem Boden 1a. Im Bodenbereich des Fermenters 1 schließt sich eine Bodenleitung 11 oder 12 an die Vertikalleitung 10 an.

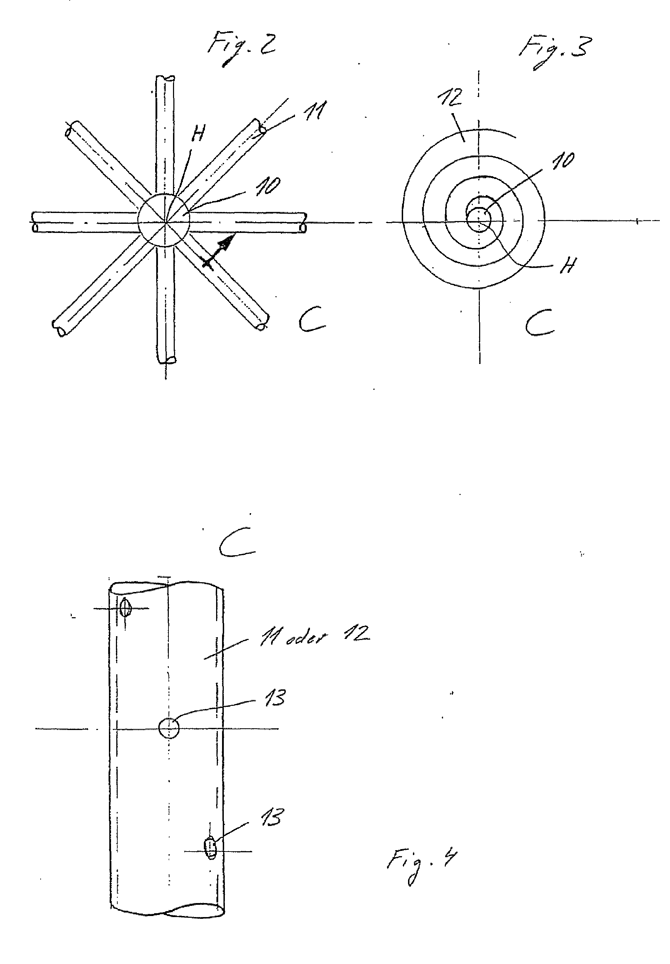

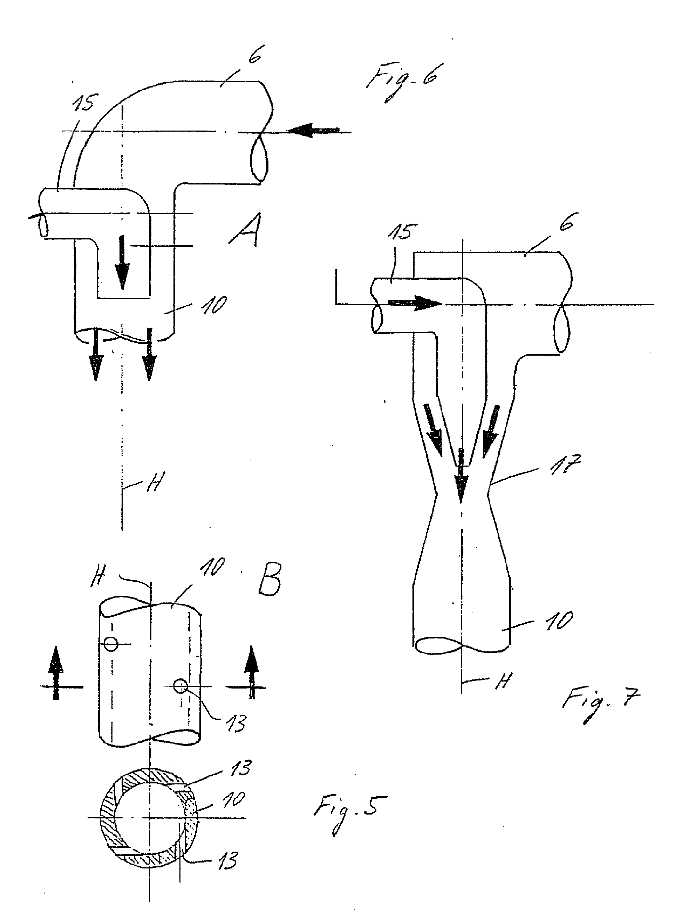

Figur 2 zeigt die nach einem ersten Ausführungsbeispiel gebildete Bodenleitung 11 und Figur 3 zeigt die nach einem zweiten Ausführungsbeispiel gebildete Bodenleitung 12. Eine der beiden Bodenleitungen 11 und 12 bedeckt den gesamten Boden des Fermenters 1.

Die Bodenleitung 11 besteht aus mehreren, vorzugsweise wenigstens sechs, im Ausführungsbeispiel aus acht Leitungsästen, die in radial nach außen von der zentralen Vertikalleitung 10 abzweigen und in einer regelmäßigen Winkelteilung angeordnet sind. Die Vertikalleitung 10 bildet einen zentralen Verbindungsknoten, durch den das Biogas in die Leitungsäste der Bodenleitung 11 strömen. Die Enden der Leitungsäste sind verschlossen. Über die gesamte Länge der Leitungsäste ist allerdings eine Vielzahl von feinen Lochdüsen verteilt angeordnet. Die Leitungsäste sind kreisrunde Rohre, vorzugsweise aus Kunststoff, die zur Bildung der Lochdüsen perforiert sind.

Die in Figur 3 gezeigte Bodenleitung 12 ist ein einziger flexibler Schlauch, der spiralig in mehreren Windungen um die Hochachse H gewunden ist. Das stromaufwärtige, innere Ende der Bodenleitung 12 ist an die Vertikalleitung 10 angeschlossen, ihr stromabwärtiges, äußeres Ende ist verschlossen. Über die gesamte Länge der Bodenleitung 12 ist ebenfalls eine Vielzahl feiner Lochdüsen verteilt angeordnet. Der radiale Abstand zwischen benachbarten Windungen beträgt etwa 40 cm, gemessen zwischen den Windungen der Mittellängslinie des Schlauchs. Die äußerste Windung hat zur Seitenwand einen Abstand von höchstens 50 cm, vorzugsweise höchstens 30 cm, gemessen gegen eine umlaufend runde Seitenwand 1b oder zu einem mittleren Bereich jeder Seite eines polygonalen Fermenters 1.

Figur 4 zeigt einen Abschnitt eines der Leitungsäste der Bodenleitung 11 und steht gleichermaßen auch für die als Schlauch gebildete Bodenleitung 12. Die beiden alternativen Bodenleitungen 11 und 12 sind wie bereits gesagt mit einer Vielzahl von feinen Lochdüsen 13 versehen, die je über die gesamte Länge jedes der Leitungsäste bzw. des Schlauchs in feiner Verteilung angeordnet sind. So können die Lochdüsen 13 insbesondere in Abständen aus dem Bereich von 5 bis 50 cm, vorzugsweise in regelmäßigen Abständen, längs der Bodenleitung 11 oder 12 angeordnet sein. In der Bodenleitung 11 oder 12 sollte in jedem Längenabschnitt von 50 cm Länge je wenigstens eine der Lochdüsen 13 vorgesehen sein. Vorzugsweise ist wenigstens eine Düse 13 in jedem Längsabschnitt von 20 cm vorgesehen. Die Düsen 13 sind über den gesamten Umfang der Bodenleitung 11 oder 12 verteilt, wenigstens sind Düsen 13 jedoch in einem Umfangsbereich der Bodenleitung 11 oder 12 angeordnet, der nach oben in das Ferment weist. Die Austrittsachsen der Düsen 13 weisen in Umfangsrichtung der jeweiligen Bodenleitung 11 oder 12 winklig zueinander, so dass das aus den Düsen 13 tretende Biogas eine Verwirbelung des Ferments und intensive Durchmischung bewirkt. Im Ausführungsbeispiel weisen die Austrittssachsen der Düsen 13 einfach radial zu einer zentralen Längsachse der Leitungsäste der Bodenleitung 11 oder einer gekrümmten, zentralen Mittellinie der Bodenleitung 12, Benachbarte Austrittsachsen sind ferner zueinander geneigt. Die Austrittsachsen können jedoch in einer Abwandlung die betreffende Längsachse oder Mittellinie je in einem Abstand kreuzen und die austretenden Gasstrahlen so gerichtet sein, dass um die Längsachse oder Mittellinie ein Drehimpuls erzeugt wird.

Figur 5 zeigt einen Längenabschnitt und einen Querschnitt der Vertikalleitung 10. Die Vertikalleitung 10 ist über ihre gesamte in das Ferment eingetauchte Länge ebenfalls mit feinen Lochdüsen 13 versehen. Beispielhaft sind die Düsen 13 mit Austrittsachsen dargestellt, die die zentrale Längsachse der Vertikalleitung 10 je in einem Abstand kreuzen, so dass um diese zentrale Längsachse, die gleichzeitig auch die zentrale Hochachse des Fermenters 1 bildet, im Ferment ein Drehimpuls erzeugt wird. Im Ausfnhrungsbeispiel sind sie tangential gerichtet. In einer Abwandlung können die Austrittsachsen der Düsen 13 aber auch einfach radial zu der Längsachse weisen. Die Vertikalleitung 10 ist im Ausführungsbeispiel als steifes Rohr, vorzugsweise Kunststoffrohr, gebildet. In einer Abwandlung kann sie insbesondere bei Verwendung der Bodenleitung 12 wie diese als flexibler Schlauch gebildet sein und die Bodenleitung 12 in einem Stück einfach verlängern. Die Düsen 13 sind längs der Vertikalleitung 10 wie eine Perlenkette angeordnet, die sich um die zentrale Längsachse windet. In der Bodenleitung 11 oder 12 sind die Düsen 13 vorzugsweise in gleicher Weise angeordnet.

Die Leitungsäste der Bodenleitung 11 und der Schlauch der Bodenleitung 12 haben einen kreisrunden Innenquerschnitt und vorzugsweise auch einen kreisrunden Außenquerschnitt. Der Innendurchmesser beträgt jeweils 3 cm. Die Vertikalleitung 10 ist ebenfalls ein kreisrundes Rohr oder Schlauch. Bei Verwendung der Bodenleitung 11 ist ihr Innendurchmesser allerdings entsprechend dem erforderlichen Durchsatz gegenüber den Innendurchmessern der Leitungsäste 11 vergrößert. Bei Verwendung der Bodenleitung 12 kann der Innendurchmesser der Vertikalleitung 10 dem der Bodenleitung 12 entsprechen. Die Düsen 13 sind Lochdüsen mit kreisrundem Düsenquerschnitt. Sie werden durch Perforation der Vertikalleitung 10 und der Bodenleitung 11 oder 12, d.h. als einfache Bohrungen erhalten. Der Durchmesser der Düsen 13 beträgt 1 bis 1.2 mm.

In der Zuführleitung 6 sind ein Kompressor 7 und stromabwärts von dem Kompressor 7 ein als Druckregelventil gebildetes Einstellglied 8 angeordnet. Der Kompressor 7 fördert das Biogas aus dem Gasspeicher 2, und das nachgeordnete Einstellglied 8 sorgt für die Einhaltung eines konstanten Drucks im Einleitsystem. Als günstig hat sich ein Überdruck von 1 bar gegenüber der Fermentsäule erwiesen, d.h. das Biogas wird mit 1 bar Überdruck durch die Düsen 13 der Bodenleitung 11 oder 12 in das Ferment geblasen.

In Figur 1 ist mit den Bezugszeichen 14 ein Gestell bezeichnet, in dem die Bodenleitung 11 oder 12 gehalten ist. Das Gestell 14 dient bei Verwendung einer flexiblen Bodenleitung wie der bevorzugten Bodenleitung 12 dazu, die Bodenleitung 12 in der Spiralform zu halten. In sämtlichen Ausführungen der Bodenleitung dient ein solches Gestell 14 insbesondere dazu, die betreffende Bodenleitung in einem Abstand über dem Boden zu halten, um auch den Boden des jeweiligen Fermenters mit dem Biogas zu beaufschlagen. Die Halterung in einem Gestell erleichtert ferner die Verlegung der Bodenleitung bereits außerhalb des Fermenters und das anschließende Einbringen in den Fermenter. Die Bodenleitung 11 oder 12 ist in dem Gestell 14 vorzugsweise nur kraft- und formschlüssig gehalten. Das Gestell 14 umfasst hierfür an den Außenquerschnitt der Bodenleitung 11 oder 12 angepasste Aufnahmen, in die die Bodenleitung 11 oder 12 eingelegt und in denen die Bodenleitung 11 oder 12 gehalten ist. Vorteilhafterweise ist das Gestell 14 ein Verbund aus in der Draufsicht auf den Boden 1a vorzugsweise schlanken Streben, in denen die Aufnahmen für die Bodenleitung 11 oder 12 nach oben offen geformt sind. Das Gestell 14 umfasst vorteilhafterweise Standfüße, so dass es auf den Standfüßen ruht, im übrigen aber ein Stück weit von dem Boden 1a freigestellt ist. Ein Teil der Aufnahmen für die Bodenleitung 11 oder 12 kann nach unten, d.h. zum Boden 1a hin, offen sein, um die Bodenleitung 11 oder 12 im Gestell 14 sowohl nach unten als auch nach oben zu sichern. Die Aufnahmen umgeben die eingelegten Leitungsäste der Bodenleitung 11 oder die Bodenleitung 12 um etwas mehr als 180°. Vorzugsweise sind die Aufnahmen etwas mehr als halbrund. Die Bodenleitung 11 oder 12 ist in die Aufnahmen eingeklemmt.

Um die Bodenleitung 11 oder 12 auf dem Boden 1a zu verlegen, wird sie in das Gestell 14 eingeklippt und mitsamt des Gestells 14 in den Fermenter 1 nach unten bis auf den Boden 1a abgelassen. Das Gestell 14 und darauf die Bodenleitung 11 oder 12 ruhen somit auf dem Boden 1a des Fermenters 1. In Figur 1 hält das Gestell 14 die Bodenleitung 11 oder 12 in einem geringen Abstand über den Boden, wobei dieser Abstand im Zentimeterbereich liegt.

Die Bodenleitung 11 oder 12 erstreckt sich über die gesamte Bodenfläche. Insbesondere die spiralig gewundene Bodenleitung 12 ermöglicht eine über dem Boden flächendeckende Beaufschlagung des Ferments mit Biogas. Aufgrund der vielen feinen Gasstrahlen wird eine besonders intensive Durchmischung erzielt, die durch die nicht parallele Ausrichtung der Düsen noch verstärkt wird. Die Durchmischung wird durch das Ausblasen auch im Bereich der Vertikalleitung 10 noch intensiviert.

Das Biogas kann über das Einleitsystem kontinuierlich, d.h. ständig in das Ferment eingeblasen werden oder aber nur zeitweise, periodisch oder nur nach Bedarf, um das Ferment intensiv zu durchmischen und anschließend wieder sich selbst zu überlassen. Falls ein periodischer Taktbetrieb oder ein Betrieb nur jeweils nach Bedarf durchgeführt werden soll, kann das Einleitsystem auch noch eine hierfür geeignete Steuerung umfassen. Vorteilhaft kann ferner auch sein, wenn der Einblasdruck des Biogases variiert werden kann. Das Einleitsystem kann auch um eine hierfür geeignete Steuerung ergänzt sein.

Das Einleitsystem kann auch für die Einbringung des biologischen Materials genutzt werden.

Figur 6 zeigt eine Substratzuführung 15, durch die biologisches Material, das im Fermenter 1 abgebaut werden soll, in das Einleitsystem eingeleitet wird. Anstatt nur das Biogas einzublasen wird in dieser Modifikation ein Gemisch aus flüssigem Substrat und Biogas in das Ferment eingespritzt. Das eingeleitete Substrat aus biologischem Material muss allerdings von einer Konsistenz sein, die ein Einspritzen erlaubt. Falls Substrat gemeinsam mit dem Biogas eingespritzt werden soll, haben die Düsen 13 einen vergrößerten Düsenquerschnitt, vorzugsweise einen Düsenquerschnitt von 7 bis 15 mm², im Falle eines bevorzugt kreisrunden Querschnitts hat dieser einen Durchmesser vorzugsweise aus dem Bereich von 3 bis 4 mm. Das Substrat wird mittels einer Pumpe durch die Substratzuführung 15 in die Vertikalleitung 10 gefördert und vermischt sich dort mit dem Biogas.

Figur 7 zeigt eine modifizierte Beimischung von Substrat. Die Substratzuführung 15 ist wie im Beispiel der Figur 6 wieder in die Vertikalleitung 10 geführt. Allerdings ist in der Vertikalleitung 10 eine Venturidüse 17 gebildet, in die die Substratzuführung 15 mündet. Das Biogas wird durch die Venturidüse 17 in Richtung auf die Bodenleitung 11 oder 12 zu beschleunigt und saugt das Substrat an, unterstützt also die Substratpumpe. Bei einem entsprechenden Unterdruck im Bereich der Venturidüse 17 kann auf eine Substratpumpe auch verzichtet werden, das Einleitsystem übernimmt dann die Substratförderung alleine.

### Bezugszeichen:

- 1: Fermenter
- 1a: Boden
- 1b: Seitenwand
- 1c: Decke
- 2: Gasspeicher
- 3: Verbindungsleitung
- 4: Abscheider
- 5: Abführleitung
- 6: Zuführleitung
- 7: Kompressor
- 8: Einstellglied
- 9: -
- 10: Vertikalleitung
- 11: Bodenleitung
- 12: Bodenleitung
- 13: Düse
- 14: Gestell
- 15: Substratzuführung
- 16: -
- 17: Venturidüse

- H: Hochachse

## Patentansprüche

1. Biogasanlage zur Erzeugung von Biogas aus einem Ferment, das wenigstens eines aus landwirtschaftlichem Abfall, Schlachtabfall, Silage oder Energiepflanzen enthält, die Biogasanlage umfassend
a) einen Fermenter (1) für einen mikrobiellen Abbau des Ferments zur Erzeugung von Biogas,
b) und ein Einleitsystem mit einer Vielzahl von Düsen (13) für das Einblasen von Biogas in das Ferment,
c) wobei die Düsen (13) über zumindest einen größeren Teil eines Bodens (1a) des Fermenters (1) verteilt angeordnet und wenigstens fünf Düsen (13) pro Quadratmeter des betreffenden Teils des Bodens vorgesehen sind.

2. Biogasanlage nach dem vorhergehenden Anspruch, wobei das Einleitsystem eine schlauch- oder rohrförmige, auf oder in einem kurzen Abstand über dem Boden (1a) angeordnete, vorzugsweise auf dem Boden abgestützte Bodenleitung (11, 12) umfasst, die einen Mantel aufweist, der mit wenigstens einem Teil der Düsen (13) versehen ist.

3. Biogasanlage nach dem vorhergehenden Anspruch, wobei die Bodenleitung (12) mehrere Schleifen umfasst, vorzugsweise spiralig oder mäanderförmig gewunden ist.

4. Biogasanlage nach einem der zwei vorhergehenden Ansprüche, wobei ein einziger Schlauch oder ein einziges Rohr die mehreren Windungen bildet,

5. Biogasanlage nach einem der drei vorhergehenden Ansprüche, wobei die Bodenleitung (11) einen Knotenbereich und mehrere davon abzweigende, vorzugsweise zumindest im Wesentlichen radial aus dem Knotenbereich weisende Leitungsäste umfasst.

6. Biogasanlage nach einem der vier vorhergehenden Ansprüche, wobei die Bodenleitung (11, 12) in einem Gestell (14) gehalten wird, das in bevorzugter Ausführung auf dem Boden aufliegt und die Bodenleitung (11, 12) wenigstens abschnittsweise, vorzugsweise über ihre gesamte Länge, in einem kurzen Abstand über dem Boden (1a) hält.

7. Biogasanlage nach einem der vorhergehenden Ansprüche, wobei das Einleitsystem wenigstens eine schlauch- oder rohrförmige, zumindest auch vertikal erstreckte Vertikalleitung (10) umfasst, die von dem Boden (1a) aufragt oder bis nahe zu dem Boden (1a) sich erstreckt, wobei ein Mantel der Vertikalleitung (10) mit wenigstens einem Teil der Düsen (13) versehen ist, und wobei in bevorzugter Ausführung die Vertikalleitung (10) mit der Bodenleitung (11, 12) nach einem der Ansprüche 2 bis 6 verbunden oder in einem Stück geformt und das Biogas durch die Vertikalleitung (10) der Bodenleitung (11, 12) zuführbar ist.

8. Biogasanlage nach einem der vorhergehenden Ansprüche, wobei das Einleitsystem eine schlauch- oder rohrförmige Leitung (10, 11, 12) umfasst, die einen Mantel aufweist, der zur Bildung wenigstens eines Teils der Düsen (13) perforiert ist, wobei vorzugsweise wenigstens ein Teil der Düsen (13) Lochdüsen sind.

9. Biogasanlage nach einem der vorhergehenden Ansprüche, wobei das Einleitsystem eine schlauch- oder rohrförmige Leitung (10, 11; 10, 12) umfasst, die einen Mantel aufweist, der mit wenigstens einem Teil der Düsen (13) versehen ist, und wobei die Düsen (13) vorzugsweise winkelig zueinander weisende Austrittsachsen aufweisen.

10. Biogasanlage nach dem vorhergehenden Anspruch, wobei die Austrittsachsen von wenigstens einer Gruppe der Düsen (13) eine gemeinsame Achse kreuzen.

11. Biogasanlage nach einem der Ansprüche 2 bis 10, wobei die Leitung (10, 11, 12) einen Strömungsquerschnitt von mindestens 7 cm² hat.

12. Biogasanlage nach einem der Ansprüche 2 bis 11, wobei die Leitung (10, 11, 12) einen Strömungsquerschnitt von höchstens 200 cm², vorzugsweise höchstens 120 cm² hat.

13. Biogasanlage nach einem der Ansprüche 2 bis 12, wobei der Mantel mit Durchbrüchen versehen ist und je einer der Durchbrüche eine der Düsen (13) bildet.

14. Biogasanlage nach einem der Ansprüche 2 bis 13, wobei die Düsen (13) in Längsrichtung der Leitung (10, 11, 12) verteilt angeordnet sind.

15. Biogasanlage nach einem der Ansprüche 2 bis 14, wobei die Düsen (13) in Umfallgsrichtung der Leitung (10, 11, 12) verteilt angeordnet sind.

16. Biogasanlage nach einem der vorhergehenden Ansprüche, wobei die Düsen (13) je einen konstanten Strömungsquerschnitt oder einen kleinsten Strömungsquerschnitt von mindestens 0.2 mm², vorzugsweise mindestens 0.5 mm² haben.

17. Biogasanlage nach einem der vorhergehenden Ansprüche, wobei die Düsen (13) je einen konstanten Strömungsquerschnitt oder einen kleinsten Strömungsquerschnitt von höchstens 10 mm², vorzugsweise höchstens 2 oder 3 mm² haben.

18. Biogasanlage nach einem der vorhergehenden Ansprüche, wobei das Einleitsystem eine Drucksteuer- oder -regeleinrichtung (7, 8) umfasst, mittels der ein Überdruck des Biogases von vorzugsweise 1 bar oder mehr einstellbar ist.

19. Biogasanlage nach einem der vorhergehenden Ansprüche, wobei das Biogas einem Gasspeicher (2) für das im Fermenter (1) erzeugte Biogas entnommen wird.

20. Biogasanlage nach einem der vorhergehenden Ansprüche, wobei eine Substratzuführung (15), die der Zuführung eines in dem Fermenter (1) mikrobiell abbaubaren Substrats dient, in das Einleitsystem mündet, wobei in bevorzugter Ausführung ein Mündungsbereich, in dem die Substratzuführung (15) in das Einleitsystem mündet, so geformt ist, dass das Substrat durch das strömende Biogas in das Einleitsystem gesogen wird.

21. Biogasanlage nach dem vorhergehenden Anspruch, wobei in dem Mündungsbereich eine Venturidüse (17) gebildet ist und die Substratzuführung (15) in einen Düsenbereich statischen Unterdrucks mündet.

22. Biogasanlage nach einem der vorhergehenden Ansprüche, wobei der Fermenter einen Innenquerschnitt von höchstens 80 m² und eine Höhe von höchstens 20 m, vorzugsweise einen Innenquerschnitt von 25 ± 10 m² und eine Höhe von 8 ± 5 m hat.

23. Verfahren zur Erzeugung von Biogas, bei dem ein Ferment in einem Fermenter (1) mikrobiell abgebaut und ein durch den Abbau gewonnenes Biogas über ein Düsenfeld mit einer Vielzahl von verteilt angeordneten Düsen (13) im Bereich eines Bodens (1a) des Fermenters (1) in das Ferment geblasen und das Ferment dadurch mit dem Biogas fein durchmischt wird.

24. Verfahren nach dem vorhergehenden Anspruch, bei dem das Biogas in unterschiedliche Richtungen in das Ferment geblasen wird, vorzugsweise von unten nach oben und in unterschiedliche Richtungen quer zur Vertikalen.

25. Verfahren nach einem der zwei vorhergehenden Ansprüche, bei dem das Biogas mit einem Überdruck von wenigstens 0.8 und vorzugsweise höchstens 1.2 bar in das Ferment geblasen wird.

26. Verfahren nach einem der drei vorhergehenden Ansprüche, bei dem das Biogas über einen größeren Teil einer Querschnittsfläche einer in dem Fermenter (1) gebildeten Säule des Ferments verteilt eingeblasen wird, wobei der größere Teil der Querschnittsfläche wenigstens 30%, vorzugsweise wenigstens 50% der gesamten Querschnittsfläche der Fermentsäule ausmacht.
